# EUROPEAN PATENT SPECIFICATION

(11) **EP 0 795 746 B1**
(45) Date of publication and mention of the grant of the patent: **16.06.2004**
(21) Application number: 97102770.1
(22) Date of filing: 20.02.1997
(51) Int. Cl.: G01N 23/04, G01N 23/10

(54) **Non-destructive X-ray inspection apparatus for food industry**
Zerstörungsfreie Röntgenstrahlungsuntersuchung für die Nahrungsmittelindustrie
Inspection par rayons X non destructive pour l'industrie alimentaire

(30) Priority: 15.03.1996 IT TO960203
(43) Date of publication of application: 17.09.1997
(60) Divisional application: 04101218.8
(73) Proprietor: Dylog Italia S.p.A., 10126 Turin (IT)
(72) Inventor: Ocleppo, Rinaldo, 12043 Canale (Cuneo) (IT)
(74) Representative: Robba, Eugenio

(56) References cited:
- EP-A- 0 375 157
- DE-A- 4 137 510
- US-A- 3 958 078
- US-A- 4 392 237
- US-A- 5 442 672

## Description

The present invention deals with a non-destructive X-ray inspection apparatus for food industry, particularly for glass vessels and/or metal cans.

It is known that, in the vast majority of cases, non-destructive X-ray inspection for glass vessel in food industry is carried out by single devices, located on one side of the row of said vessels to be inspected; said devices being substantially equipped with an emitter and a sensor between which the row of said vessels to be inspected moves.

Similarly known is the fact that with such single devices a 90-95% inspection is obtained of the product contained therein, said deficiency being due to the shadow area projected by the concavity that is present, more or less importantly, in the vessel bottom.

To be more precise, refer to a glass vessel containing jam or a similar product; the impairing concavity is the one on the bottom of these vessels; this projection overlaps the contaminating agents hiding them and making their recognition impossible.

In many cases this result has been deemed, wrongly, enough, because an inspection valid only at 95% created a lot of problems, which it was attempted to solve through a few solutions, that proved however rather complicated and costly and not satisfactory enough.

Among the above solutions, mention can be made of a 45°-slant on the vertical emitter pipe plan, solution that only generated another shadow area in another point with respect to the traditional inspection apparatus placed on one side; moreover, though mitigating the darkening, it did not solve the problem, often due to high vessel tolerances. Furthermore, it cannot be applied to cans.

Another solution being studied in the United Kingdom provides two inspection apparata mutually placed at 90° on the vertical plan.

This latter solution seems to benefit from a partial reduction of shadow areas, but it uses two inspection apparata and the system overall dimensions are rather big; the cost for the two apparata, their maintenance and the like are important cost elements that cannot be overlooked.

Document DE 41 37 510 illustrates a scanning system for investigating test object passing on a transport path for unauthorised contents. The system contains a source of radiation emitting two fan-shaped beams toward two corresponding linear sensors. The angle between the plane of at least one beam and the transport direction differs from 90 deg. A signal processing device process the signals of the sensors in order to provide a tridimensional view of the scanned object.

US 3 958 078 refers to an X-ray inspection apparatus for scanning articles containing food products. One of the embodiment illustrated provides for the presence of two independent X-ray sources coupled to corresponding sensors, the two scanning directions being mutually arranged at 90 deg.

US 5 442 672 illustrates an apparatus for reconstructing a tridimensional image of an object. This apparatus provides as well the presence of two independent X-ray sources coupled to corresponding sensors, mutually arranged at 90 deg.

Patent application EP 0 375 157 relates to an on-line X-ray inspection system comprising a source of X-rays providing a very thin beam of rays of general fan shape and, opposite to the X-ray source, a detector means in the form of a linear array of photo-sensitive diodes.

Object of the present invention is solving the above-mentioned problem of removing the shadow area when inspecting packaged vessels, realizing an improved, simple and practical apparatus that allows easily obtaining the provided object of a complete inspection of sealed vessels.

The inspection apparatus of the present invention is composed of a static structure comprising two modular units mutually placed at 90° and placed at 45° with respect to a feeding line for vessels to be inspected; a first one of said two units being equipped with a semi-panoramic emitter and an X-ray linear sensor, a second one of said two units being equipped only with a sensor, equal to said sensor of said first unit; said inspection structure with a single emitter and two sensors realizing a structure able to inspect 100% of a product contained in said vessels.

The advantages of the apparatus of the invention are as follows:
- modularity: therefore, with the same performance a starting price (for the single module) is much less than the standard price, thus widening product markets;
- with two sensors a simultaneous failure thereof is statistically impossible, so that a user can go on working even in case one of the two sensors fails (inspecting 95% of the product);
- there are practically no problems with installation related costs;
- there are no costs for mechanical spare parts, being the structure substantially static with obvious maintenance advantages;
- there are no problems of space, thus complying with the food industry needs whose lines are almost always full and without spaces for apparata not provided for when designing.

Further features and advantages will appear from the following detailed description of an embodiment of the invention, with reference to the accompanying drawings, provided as a non limiting example, in which:
- Fig. 1 is a schematic plan view of a prior art inspection apparatus, placed at 90° with respect to the line of vessels to be inspected;
- Fig. 2 is a schematic plan view of a prior art inspection apparatus, placed at 45° with respect to the line of vessels;
- Fig. 3 is a schematic sectional view of the apparatus in Fig. 1;
- Fig. 4 is a schematic plan view of an inspection apparatus according to a first embodiment of the invention;
- Fig. 5 is a schematic plan view of an inspection apparatus ;
- Fig. 6 is a section of a glass vessel with the typical bottom concavity.

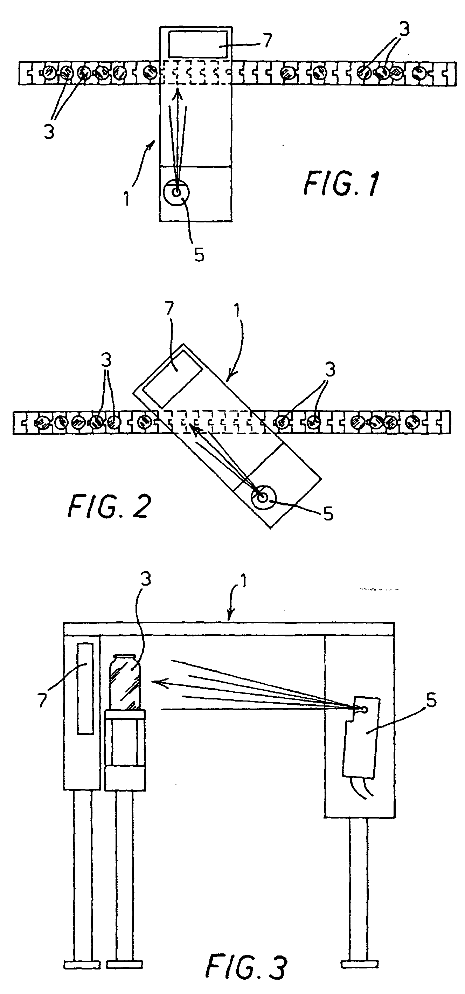

As appears from Figures 1 and 3, prior art inspection apparata are composed of a structure 1 bridging a row on glass vessels 3 to be examined that move thereunder.

This structure includes a X-ray emitter 5 and a sensor 7, that collects rays emimtted by the emitter 5 and displays what has been inspected.

If a glass vessel is taken into account, it can be clearly seen how the concavity 9 provided on the bottom of said vessel 3 impairs a complete inspection of the product in the vessel; should this concavity or internal projection 9 be missing, obviously there would be no problems.

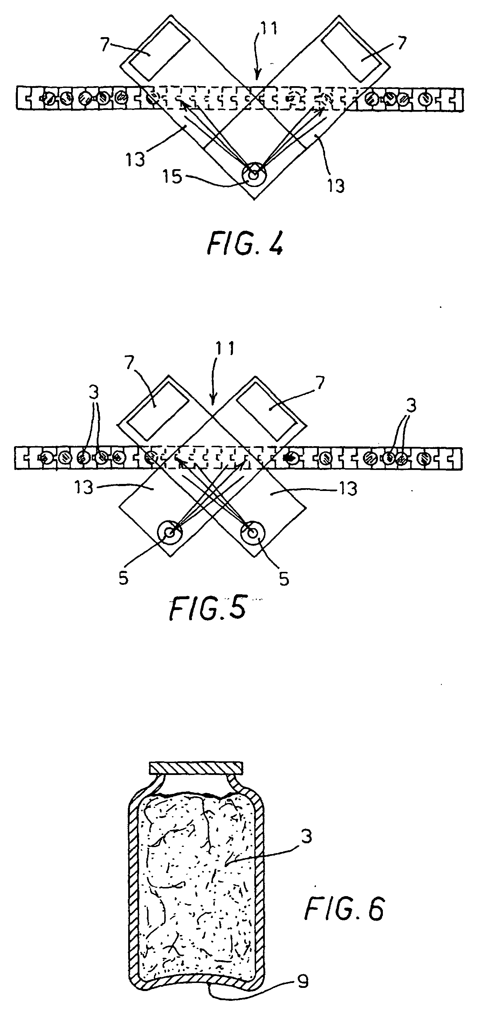

With the inspection apparatus of the invention shown in Fig. 4, instead, a static structure 11 is provided, composed of two modular units 13 placed at 90° one to the other and at 45° with respect to the feeding line of vessels 3 to be inspected.

One of these two units 13 is equipped with a semi-panoramic emitter 15 and with a normal sensor 7, while the other unit is equipped only with the sensor 7 equal to the one in the first unit.

The semi-panoramic emitter 15 is a source of radiation capable of emitting an X-ray beam having an aperture, on an horizontal plane, of at least 90° in order to cover both sensors 7.

In the embodiment of the inspection apparatus shown in Fig. 5 which is not part of the invention both units 13 are each equipped with a standard emitter 5 and a sensor 7.

It is clear that in this way shadow areas created by the concavity 9 of the vessels to be inspected, are removed, since the area not detected by a sensor will be detected by the other sensor at 90° with respect to the first one.

It is clear that, in case of failure of one of these sensors 7, a user can go on working, if he deems it adequate, with only one of them, always getting a 95% inspection as was the case in prior art.

## Claims

1. Non-destructive X-ray inspection apparatus for glass vessels and/or cans for food industry, comprising a feeding line for transporting said vessels and/or cans (3) through said apparatus and two scanning units (13) mutually placed at 90°, **characterised in that**:
- a first unit (13) is equipped with emitter means (15) for emission of an X-ray beam having an aperture, on an horizontal plane, of at least 90°, and with sensor means (7) facing said emitter means (15);
- a second unit (13) is equipped with sensor means (7), equal to said sensor means in said first unit and facing said emitter means (15);
wherein said first and second units are modular units placed in relation to each other in such a manner that X-rays collected by said sensor means in said first modular unit and X-rays collected by said sensor means in said second modular unit are both emitted from said emitter means (15) and form an angle of 90° in relation to each other, and wherein said first and second modular units are placed in relation to said feeding line in such a manner that X-rays collected by said sensor means in said first modular unit and X-rays collected by said sensor means in said second modular unit intersect said feeding line at an angle of 45°, so that said vessels and/or cans passing on said feeding line are inspected along two perpendicular directions.

2. Inspection apparatus according to claim 1, wherein said apparatus is adapted to be used as a normal unitary structure for a 95% inspection using a single one of said modular units at 45° with respect to the line of vessels (3) to be examined, using said emitter means (15) and only one of said sensor means (7).

3. Inspection apparatus according to claim 1, wherein said apparatus with a single emitter and two sensors provides the capability to inspect 100% of a product contained in said vessels and/or cans.

4. Inspection apparatus according to claim 1, wherein said emitter means (15) comprises a semi-panoramic emitter.

## Patentansprüche

1. Einrichtung zur zerstörungsfreien Röntgenstrahluntersuchung für Glasbehälter und/oder Dosen für die Nahrungsmittelindustrie, die eine Förderstrecke für den Transport der Behälter und/oder Dosen (3) durch die Einrichtung sowie zwei Scannereinheiten (13) umfasst, die um 90° zueinander versetzt angeordnet sind,
**dadurch gekennzeichnet, daß**: eine erste Einheit (13) mit Sendeelementen (15) zur Abstrahlung eines Röntgenstrahls mit einer Öffnung von mindestens 90° in einer Horizontalebene ausgestattet ist, sowie Sensorelementen (7), die den Sendeelementen (15) gegenüberliegen;
eine zweite Einheit (13) mit einem Sensorelement (7), das dem Sensorelement der ersten Einheit gleichartig ist und das dem Senderelement (15) gegenüberliegt;
wobei die erste und zweite Einheit modulare Einheiten sind, die derart zueinander in Bezug gesetzt sind, daß die vom Sensorelement in der ersten modularen Einheit aufgenommenen Röntgenstrahlen und die vom Sensorelement in der zweiten modularen Einheit aufgenommenen Röntgenstrahlen beide vom Senderelement (15) emittiert werden und einen Winkel von 90° zueinander bilden und wobei die erste und die zweite modulare Einheit in Bezug auf die Förderstrecke so plaziert sind, daß die vom Sensorelement in der ersten modularen Einheit aufgenommenen Röntgenstrahlen und die vom Sensorelement in der zweiten modularen Einheit aufgenommenen Röntgenstrahlen die Förderstrecke unter einem Winkel von 45° schneiden, so daß die die Förderstrecke passierenden Behälter und/oder Dosen längs zweier lotrechter Richtungen untersucht werden.

2. Untersuchungseinrichtung nach Anspruch 1, wobei die Einrichtung angepaßt wird, um als normale Einheitsbaueinheit zur 95%-igen Prüfung eingesetzt zu werden unter Verwendung einer der modularen Einheiten unter 45° in Bezug auf die zu untersuchende Behälterkolonne (3), wobei das Senderelement (15) und nur eines der Sensorelemente (7) verwendet wird.

3. Untersuchungseinrichtung nach Anspruch 1, wobei die Einrichtung mit einem einzigen Sender und zwei Sensoren die Fähigkeit beinhaltet, 100 % eines in den Behältern und/oder Dosen enthaltenen Produkts zu untersuchen.

4. Untersuchungseinrichtung nach Anspruch 1, wobei das Senderelement (15) einen halbpanoramischen Sender aufweist.

## Revendications

1. Appareil d'analyse non destructive par les rayons X destiné à des enceintes et/ou des récipients de verre pour l'industrie alimentaire, comportant une ligne d'alimentation pour transporter lesdites enceintes et/ou récipients (3) à travers ledit appareil et deux unités de balayage (13) placées mutuellement à 90°, **caractérisé en ce que** :
- une première unité (13) est dotée de moyens émetteurs (15) pour une émission d'un faisceau de rayons X, présentant une ouverture, dans un plan horizontal, d'au moins 90°, et de moyens détecteurs (7) faisant face aux dits moyens émetteurs (15) ;
- une seconde unité (13) est dotée de moyens détecteurs (7), équivalents aux dits moyens détecteurs de ladite première unité et faisant face aux dits moyens émetteurs (15) ;
dans lequel lesdites première et seconde unités sont des unités modulaires placées en relation l'une par rapport à l'autre de telle sorte que les rayons X collectés par lesdits moyens détecteurs de la première unité modulaire et les rayons X collectés par lesdits moyens détecteurs de ladite seconde unité modulaire soient tous deux émis à partir desdits moyens émetteurs (15) et forment un angle de 90° les uns par rapport aux autres, et dans lequel lesdites première et seconde unités modulaires sont placées en relation avec ladite ligne d'alimentation de telle manière que les rayons X collectés par lesdits moyens détecteurs de ladite première unité modulaire et les rayons X collectés par lesdits moyens détecteurs de ladite seconde unité modulaire coupent ladite ligne d'alimentation sous un angle de 45°, de façon que lesdites enceintes et/ou lesdits récipients passant sur ladite ligne d'alimentation soient examinés suivant deux directions perpendiculaires.

2. Appareil d'analyse selon la revendication 1, dans lequel ledit appareil est adapté pour être utilisé comme une structure unitaire normale pour un examen à 95% en utilisant une seule desdites unités modulaires à 45° par rapport à la ligne des enceintes (3) à examiner, en utilisant lesdits moyens émetteurs (15) et l'un seulement desdits moyens détecteurs (7).

3. Appareil d'analyse selon la revendication 1 dans lequel ledit appareil comportant un seul émetteur et deux détecteurs fournit la capacité d'analyser 100% d'un produit contenu dans lesdites enceintes et/ou lesdits récipients.

4. Appareil d'analyse selon la revendication 1, dans lequel lesdits moyens émetteurs (15) comprennent un émetteur semi-panoramique.
